# EUROPÄISCHE PATENTSCHRIFT

(11) **EP 2 340 769 B1**
(45) Veröffentlichungstag und Bekanntmachung des Hinweises auf die Patenterteilung: **30.01.2013**
(21) Anmeldenummer: 10197286.7
(22) Anmeldetag: 29.12.2010
(51) Int. Cl.: A61F 2/00, A61B 17/00, A61B 17/04, A61B 17/06

(54) **Implantat, insbesondere zur Behandlung von Harninkontinenz, Verfahren zu dessen Herstellung und chirurgisches Set**
Implant, in particular for treating urinary incontinence, method for producing same and surgical set
Implant, notamment pour le traitement de l'incontinence uréique, son procédé de fabrication et ensemble chirurgical

(30) Priorität: 31.12.2009 DE 102009060802
(43) Veröffentlichungstag der Anmeldung: 06.07.2011
(73) Patentinhaber: Aesculap AG, 78532 Tuttlingen/Donau (DE); ITV Denkendorf Produktservice GmbH, 73770 Denkendorf (DE)
(72) Erfinder: Odermatt, Erich, 8200, Schaffhausen (CH); Weis, Christine, 8190, Sant Cugat del Vallés (ES); Müller, Erhard, 70565, Stuttgart (DE); Schmees, Hans-Gerd, 72827, Wannweil (DE)
(74) Vertreter: Patentanwälte Ruff, Wilhelm, Beier, Dauster & Partner

(56) Entgegenhaltungen:
- EP-A1- 0 108 171
- WO-A1-98/49967
- WO-A1-02/078552
- WO-A2-02/078571
- US-A1- 2003 130 670

## Beschreibung

Die Erfindung betrifft ein Implantat, welches sich insbesondere zur Behandlung der Harninkontinenz eignet, ein Herstellungsverfahren für das Implantat sowie ein chirurgisches Set zur Behandlung der Harninkontinenz.

Die Harninkontinenz, d.h. der unwillkürliche, unfreiwillige Harnverlust, ist eine vor allem mit zunehmendem Alter bei Frauen und Männer weit verbreitete Erkrankung. Allein in Deutschland leiden derzeit etwa 6 Millionen Menschen an einer Harninkontinenz.

Die Medizin kennt mehr als ein halbes Dutzend Erscheinungsformen der Harninkontinenz. Die wichtigsten Erscheinungsformen sind die Stress-, Drang- und die Überlaufinkontinenz.

Die Stress- oder Belastungsinkontinenz tritt vor allem bei Frauen auf. Hierbei kommt es zu einem ungewollten Urinverlust bei körperlichen Belastungen, insbesondere bei Lachen, Husten, Niesen oder körperlichen Übungen. Sie resultiert aus einer Schwäche des Harnröhren-Schließmuskels, der aufgrund einer Lockerung der Muskeln des Perineums und/oder aufgrund eines intrinsischen Schließmuskeldefektes (ISD) nicht länger in der Lage ist, die Blase abzudichten.

Von der Dranginkontinenz sind vor allem ältere Menschen betroffen. Die Ursache liegt in einer Überaktivität oder Überempfindlichkeit der Blase. Einerseits werden die Signale über den Füllungszustand der Blase im Rückenmark nicht richtig verarbeitet, anderseits ist die Blase nicht mehr in der Lage, sich "auf Befehl" vollständig zu entleeren. Daraus entsteht ein Missverhältnis zwischen überfallartigem Harndrang mit Urinverlust und der Unfähigkeit, sich willentlich zu "erleichtern". In den Frühstadien spricht man auch von einer "Reizblase".

Die Überlaufinkontinenz tritt vor allem bei Männern auf. Sie ist gekennzeichnet durch einen unfreiwilligen, tropfenweisen Urinverlust bei stark gefüllter Harnblase. Häufiges Wasserlassen mit geringer Urinmenge (sogenanntes Miktionsvolumen) ist die Regel. Deshalb bleiben große Restharnmengen zurück. Ursachen sind Abflussbehinderungen im Bereich des Blasenausgangs bzw. des Blasenhalses oder der Harnröhre. Da die Prostata unterhalb der Harnblase des Mannes liegt und die Harnröhre beim Austritt aus der Blase umschließt, können Vergrößerungen der Prostata die Harnröhre abdrücken. Als Folge hiervon kann der Blasenmuskel nicht mehr genug Kraft aufwenden, um die Blase zu entleeren, wodurch sich die Blase immer weiter füllt. Erst wenn der Füllungsdruck der Blase den Verschlussdruck übersteigt, geht unfreiwillig Urin ab. Deswegen ist das Vorliegen einer benignen Prostatahyperplasie oder eines Prostatakarzinoms hänfig die Ursache für das Auftreten einer Überlaufinkontinenz. Nach einer (teilweisen) Resektion der Prostata (sogenannte Prostatatektomie) können auch Männer gehäuft unter Stressinkontinenz leiden. Die Stressinkontinenz kann dabei - wie bei Frauen - auf einer intrinsischen Schließmuskelschwäche (ISD) beruhen.

In der Regel wird zur Behandlung der Stressinkontinenz ein künstlicher, hydraulischer Urinsphinkter implantiert. Derartige Sphinkter sind jedoch zum Einen aufwendig und teuer in ihrer Herstellung. Zum Anderen sind sie häufig Ursache für Gewebeerosionen und/oder Infektionen. Das kann zu postoperativen Komplikationen führen und insbesondere chirurgische Re-Interventionen erforderlich machen.

Bereits in den sechziger Jahren des letzten Jahrhunderts wurde die sogenannte Zügelplastik-Technik entwickelt. Bei dieser Technik wird im Bauchraum ein Band fixiert, das unter der Harnröhre hindurchgeführt wird, um diese zusammen mit dem Blasenhals etwas anzuheben, was insbesondere in Fällen einer Blasensenkung hilfreich ist. Besonders bewährt hat sich die minimalinvasive Einbringung eines Implantates, das beispielsweise bei weiblichen Patienten durch die Vaginalwand hindurchgeführt und als U-förmige Schlinge, die die Harnröhre untergreift, im Bauchraum platziert wird, wobei die freien Enden der Schlinge in der Bauchdecke frei enden. Das Band wird durch einwachsendes Bindegewebe verankert. Solche Bänder und die zugehörigen Instrumente zur Einbringung in den Unterleib sind beispielsweise in den Druckschriften WO 90/0 37 66, WO 96/0 65 67, WO 97/134 65 und WO 2001/0 30 2 46 beschrieben.

Aus der WO 02/078571 A2 sind Implantate zur Behandlung von Harninkontinenz bekannt sowie Vorrichtungen, um diese im Bereich des retropubischen Raums zu platzieren und mit der endopelvinen Faszie zu verankern. Die Implantate können mit Hilfe eines resorbierbaren Nahtmaterials mit chirurgischen Einführinstrumenten verbunden werden.

Die US 2003/0130670 A1 offenbart textile Harninkontinenzbänder, die mit einem Dilator im Körper eines Patienten platziert werden können. Ein mit dem Inkontinenzband verbundener Zugfaden erleichtert dem Chirurgen die Platzierung des Bandes.

In der WO 02/078552 A1 werden Prolapsnetze beschrieben, die eine Öffnung mit einem um diese Öffnung angeordneten Zugfaden aufweisen. Bei Zugbelastung des Fadens kommt es zu einer Verengung der Öffnung und damit zu einer Verkürzung des Prolapsnetzes, wodurch dessen Platzierung im Körper des Patienten erleichtert wird.

Die EP 0108171 A1 beschreibt gewebte Transplantatmaterialien, die derart ausgestaltet sind, dass sie beidseitig den Einwuchs von Gewebe erleichtern. Beispielsweise kann das Material in Form eines Doppelvelours vorliegen, das zum einen einer vorzeitigen Blutgerinnung entgegenwirkt und zum anderen den Gewebeeinwuchs fördert.

Die WO 98/49967 A1 beschreibt eine Stoffprothese zur Gewebereparatur, die eine mikroporöse Barrierelage sowie eine poröse, den Gewebeeinwuchs fördernde Lage umfasst. Die einzelnen Lagen können beispielsweise eine Atlas-Konstruktion aufweisen.

Zur Verbindung eines Harninkontinenzbandes mit einem Einführinstrument, kommen in der Regel sogenannte Adapter zum Einsatz. Hierbei handelt es sich um gewöhnlich aus Kunststoffmaterialien bestehende Verbindungsstücke, welche in Richtung des Einführinstrumentes häufig eine konische Verjüngung aufweisen. Durch die konische Verjüngung wird der durch das Einführinstrument geformte Stichkanal dilatiert, damit das Harninkontinenzband möglichst reibungsarm hindurchgleiten kann. Nachteilig hierbei ist, dass der Adapter in einem separaten Fertigungsschritt hergestellt, an das Harninkontinenzband spezifisch angepasst und mit dem Harninkontinenzband verbunden werden muss. Sehr häufig wird das Verbinden von Harninkontinenzband und Adapter vom Chirurgen selbst durchgeführt, was die Handhabung entsprechender Harninkontinenzband-Adapter-Kombinationen erschwert. Hinzu kommt, dass die Adapter in der Regel aus eher harten Kunststoffmaterialien gebildet sind, die zu unerwünschten Gewebetraumatisierungen führen können.

Demnach liegt der vorliegenden Erfindung die Aufgabe zugrunde, ein chirurgisches Implantat bereitzustellen, welches aus dem Stand der Technik bekannte Unzulänglichkeiten vermeidet und insbesondere ohne die Verwendung von Adaptern oder Dilatatoren mit chirurgischen Einführinstrumenten verbindbar ist.

Diese Aufgabe wird erfindungsgemäß gelöst durch ein chirurgisches Implantat mit den Merkmalen des unabhängigen Anspruchs 1. Bevorzugte Ausführungsformen des Implantats sind Gegenstand der abhängigen Ansprüche 2 bis 10. Ein Verfahren zur Herstellung des Implantats ist Gegenstand des Anspruchs 11. Bevorzugte Ausführungsformen des Herstellungsverfahrens sind in den abhängigen Ansprüchen 12 bis 15 wiedergegeben. Außerdem betrifft die vorliegende Erfindung ein chirurgisches Set mit den Merkmalen des Anspruchs 16. Der Wortlaut sämtlicher Ansprüche wird hiermit durch Bezugnahme zum Inhalt der vorliegenden Beschreibung gemacht.

Bei dem erfindungsgemäßen Implantat handelt es sich um ein chirurgisches Implantat, vorzugsweise zur Anwendung bei der Behandlung von Harninkontinenz, in Form eines Bandes mit textiler Struktur, einem Mittelabschnitt und zwei Endabschnitten beidseits des Mittelabschnitts. Das bandförmige Implantat weist im Bereich zumindest eines der beiden Endabschnitte zumindest ein textil ausgebildetes bzw. textiles Befestigungsmittel zur Befestigung des Bandes an ein chirurgisches Einführinstrument, vorzugsweise an eine chirurgische Nadel, auf. In der Regel sind der Mittelabschnitt und die beiden Endabschnitte des Bandes und vorzugsweise das Band selbst streifenförmig ausgebildet.

Der Ausdruck "zumindest ein" kann im Sinne der vorliegenden Erfindung ein, zwei, drei oder eine Mehrzahl, insbesondere Vielzahl, von etwas bedeuten. So meint beispielsweise der Ausdruck "zumindest ein textil ausgebildetes Befestigungsmittel" im Sinne der vorliegenden Erfindung, dass das Band ein, zwei, drei oder mehrere textil ausgebildete Befestigungsmittel, insbesondere eine Vielzahl von textil ausgebildeten Befestigungsmitteln, aufweisen kann.

Der Ausdruck "textil ausgebildetes Befestigungsmittel" soll im Sinne der vorliegenden Erfindung möglichst umfassend verstanden werden und meint insbesondere, dass das Befestigungsmittel eine textile Struktur besitzen kann und/oder nach einem textilen Verfahren hergestellt sein kann. Die textile Struktur kann beispielsweise von einem Faden oder einem Fadenteil bzw. -abschnitt gebildet sein.

Das zumindest eine textil ausgebildete Befestigungsmittel dient mit besonderem Vorteil als Ersatz für die aus dem Stand der Technik bekannten, in der Regel aus hartem Kunststoff bestehenden und daher beim Einzug in ein biologisches Gewebe Traumatisierungen verursachenden Adapter bzw. Dilatatoren. Ein aufwendiges und umständliches Anpassen und Befestigen von Adaptern an Implantate, insbesondere Inkontinenzbänder, ist nicht länger erforderlich. Die Befestigung des Bandes an ein chirurgisches Einführinstrument erfolgt über das zumindest eine textil ausgebildete Befestigungsmittel. Abhängig von der Ausbildung des zumindest einen textilen Befestigungsmittels kann dies beispielsweise durch einen einfachen Auf- oder Einhäng- oder Verhakungsmechanismus erfolgen. Dies führt insgesamt zu einer deutlich vereinfachten Handhabung des Bandes, insbesondere in Kombination mit einem chirurgischen Instrument zur Einführung des Bandes in den Körper eines Patienten. Durch die Zugkraft, die der Chirurg während der Implantation über das chirurgische Einführinstrument auf das Band ausübt, verjüngt sich der Banddurchmesser. Dies ermöglicht einen im Wesentlichen glatten Gewebeeinzug des Bandes, so dass kaum Gewebetraumatisierungen infolge von Reibungsvorgängen, wie sie typischerweise bei der Verwendung von Adaptern oder Dilatatoren auftreten, zu befürchten sind.

Der Mittelabschnitt des Bandes ist in der Regel zur Unterstützung bzw. Anhebung einer anatomischen Struktur vorgesehen. Bevorzugt dient der Mittelabschnitt der Unterstützung des Blasenhalses und/oder der Harnröhre, insbesondere der bulbären Harnröhre. Mit besonderem Vorteil wird durch das erfindungsgemäße Band lediglich der ventrale Bereich einer Harnröhre komprimiert, so dass die Blutversorgung in den dorsalen und lateralen Bereichen hiervon unbetroffen ist. Bevorzugt wird bei einer Verwendung des Bandes als Inkontinenzband der bulbäre spongiose Muskel des Patienten intakt gelassen, so dass dieser nach Art eines "schützenden Kissens" die dahinterliegende Harnröhre vor Gewebeerosionen schützen kann.

Die Endabschnitte dienen hauptsächlich der Befestigung des Bandes im Körper eines Patienten, beispielsweise an ventrale, laterale und/oder dorsale Gewebebereiche im Bereich der Harnröhre eines Patienten. Das Band kann dabei mittels Gewebeklebern und/oder Nähten im Körper des Patienten befestigt werden. Gegebenenfalls kann es vorgesehen sein, die Endabschnitte nach Befestigung des Bandes zu kürzen.

Zumindest einer der beiden Endabschnitte, insbesondere beide Endabschnitte, besitzt in einer weitergehenden Ausführungsform ein sich im Durchmesser verjüngendes und vorzugsweise abgerundetes Ende.

In einer bevorzugten Ausführungsform weist das Band im Bereich von beiden Endabschnitten jeweils zumindest ein, insbesondere ein, zwei, drei oder mehrere, textil ausgebildetes Befestigungsmittel auf. Auf diese Weise lässt sich das Band über beide Enden jeweils an ein chirurgisches Einführinstrument, bevorzugt an eine chirurgische Nadel, befestigen. Die Befestigungsmittel im Bereich der Endabschnitte können gleich oder unterschiedlich ausgebildet sein. Geeignete Ausbildungsformen für das zumindest eine textile Befestigungsmittel werden im Folgenden eingehender erläutert.

Das zumindest eine textil ausgebildete Befestigungsmittel ist in die textile Struktur des Bandes eingebunden. Auf diese Weise wird mit besonderem Vorteil eine vorzeitige Ablösung des zumindest einen textil ausgebildeten Befestigungsmittels von dem Band, insbesondere während der Implantation des Bandes, und/oder eine postoperative Ablösung verhindert.

Das zumindest eine textil ausgebildete Befestigungsmittel kann aus der Ebene der textilen Struktur des Bandes herausragen und/oder in der Ebene der textilen Bandstruktur ausgebildet sein. Insbesondere kann es erfindungsgemäß vorgesehen sein, dass das Implantat im Bereich des einen Endabschnittes zumindest ein aus der Ebene der textilen Bandstruktur herausragendes, textil ausgebildetes Befestigungsmittel aufweist und im Bereich des anderen Endabschnittes ein in der Ebene der textilen Bandstruktur liegendes, textil ausgebildetes Befestigungsmittel aufweist.

In einer weiteren Ausführungsform ist das zumindest eine textil ausgebildete Befestigungsmittel am Rand- oder Kantenbereich des zumindest einen Endabschnittes, insbesondere der beiden Endabschnitte, ausgebildet. Die Ausbildung des zumindest einen textilen Befestigungsmittels am Rand- bzw. Kantenbereich des Bandes hat den Vorteil, dass sich eine Verbindung zwischen dem erfindungsgemäßen Implantat und einem chirurgischen Einführinstrument leichter herstellen lässt.

In einer besonders bevorzugten Ausführungsform ist das zumindest eine textil ausgebildete Befestigungsmittel schlaufen-, loop- oder schlingenförmig ausgebildet. In dieser Ausführungsform kann das zumindest eine textil ausgebildete Befestigungsmittel beispielsweise einen kreis-, O- und/oder ovalförmigen, insbesondere ellipsoiden, Verlauf besitzen.

Das zumindest eine textil ausgebildete Befestigungsmittel ist in einer weitergehenden Ausführungsform unter Ausbildung einer Schlaufe, Schlinge oder eines Loops mit dem zumindest einen Endabschnitt, vorzugsweise mit beiden Endabschnitten, verbunden.

Das zumindest eine textil ausgebildete Befestigungsmittel ist fadenförmig ausgebildet. Zumindest ein Faden ist am Aufbau der textilen Struktur des Bandes beteiligt bzw. in die textile Bandstruktur eingebunden. Bei dem zumindest einen Faden kann es sich um einen originären Bandfaden, d.h. um einen Faden, der zur Herstellung des Bandes verwendet wurde, und/oder um einen zusätzlich in die textile Struktur des Bandes eingezogenen Faden handeln. Durch den zusätzlichen Einzug eines oder gegebenenfalls von mehreren Fäden ist es nicht erforderlich, Fäden der Bandstruktur zur Ausbildung des zumindest einen textilen Befestigungsmittels zu verwenden, was gegebenenfalls zu einer Schwächung der Bandstruktur führen würde. Der zumindest eine Faden kann als Monofilament, Pseudomonofilament, Multifilament, insbesondere geflochtenes Multifilament, oder Multifilamentgarn ausgebildet sein.

In einer weitergehenden Ausführungsform umfasst das zumindest eine textil ausgebildete Befestigungsmittel zwei, drei oder mehrere Fäden, insbesondere zwei, drei oder mehrere zusätzlich in die Bandstruktur eingezogene Fäden. Dies ermöglicht mit besonderem Vorteil eine stabilere und damit sichere Befestigung bzw. Aufhängung des Bandes an ein chirurgisches Einführinstrument. In einer weiteren Ausführungsform umfasst das zumindest eine textil ausgebildete Befestigungsmittel zumindest einen Bandfaden, gegebenenfalls mehrere Bandfäden, und zumindest einen zusätzlich in die Bandstruktur eingezogenen Faden, gegebenenfalls mehrere zusätzlich in die Bandstruktur eingezogene Fäden. Bezüglich weiterer Merkmale und Einzelheiten der in diesem Absatz beschriebenen Ausführungsformen wird insbesondere auf den vorherigen Absatz Bezug genommen.

In geeigneten Ausführungsformen ist das zumindest eine textil ausgebildete Befestigungsmittel als Fadenschlinge, insbesondere Veloursschlinge und/oder Flottung, ausgebildet.

Gemäß einer weiteren Ausführungsform ist das zumindest eine textil ausgebildetes Befestigungsmittel ein strang- oder schnurförmiges Textilgebilde, insbesondere eine Kordel oder ein Geflecht, insbesondere Rund- oder Schlauchgeflecht. Die Ausbildung des zumindest einen textil ausgebildeten Befestigungsmittels als strang- bzw. schnurförmiges Textilgebilde erlaubt eine besonders sichere Aufhängung des Bandes an ein chirurgisches Einführinstrument.

Das Band weist in einer weiteren Ausführungsform zumindest im Bereich eines seiner beiden Endabschnitte, vorzugsweise im Bereich von beiden Endabschnitten, zumindest einen, insbesondere einen, aus der Bandstruktur entfernbaren Trenn- oder Separierfaden auf, der sich vorzugsweise in Längsrichtung des Endabschnittes bzw. der Endabschnitte erstreckt. Entsprechende Trenn- oder Separierfäden können, wie im nächsten Absatz beschrieben, zur Ausbildung von vorzugsweise schlitzförmigen Öffnungen dienen.

Zur Einstellung der Spannung des Mittelabschnittes beim Implantieren des Bandes kann zumindest ein Endabschnitt, vorzugsweise beide Endabschnitte, eine Öffnung aufweisen, an der die Bandstruktur vorzugsweise durchbrochen ist. Die Öffnung kann grundsätzlich kreis-, oval-, insbesondere ellipsoid-, streifen- oder schlitzförmig ausgebildet sein. Bevorzugt ist die Öffnung jedoch schlitzförmig ausgebildet. Bei der Öffnung selbst kann es sich um eine mechanisch, thermisch oder mittels eines Lasers erzeugte Öffnung handeln. Die Öffnung kann insbesondere durch Schneid- und/oder Stanzvorgänge erzeugt sein. In bevorzugten Ausführungsformen ist die Öffnung durch Entfernen, insbesondere Herausschneiden, zumindest eines in Längsrichtung zumindest eines der beiden Endabschnitte, vorzugsweise von beiden Endabschnitten, verlaufenden Trenn- oder Separierfadens gebildet.

In einer weiteren geeigneten Ausführungsform liegt das zumindest eine textil ausgebildete Befestigungsmittel verstärkt bzw. versteift vor. Zur Verstärkung bzw. Versteifung des Befestigungsmittels kann dieses mehrere Fäden umfassen und beispielsweise, wie bereits vorstehend erwähnt, als Multifilament, Multifilamentgarn, Zwirn, Kordel oder Geflecht vorliegen. In anderen Worten kann das Befestigungsmittel textil verstärkt bzw. textil versteift vorliegen. Alternativ oder in Kombination hierzu kann das zumindest eine textil ausgebildete Befestigungsmittel thermofixiert oder verschweißt, insbesondere ultraschallverschweißt, vorliegen. Beispielsweise kann das zumindest eine textil ausgebildete Befestigungsmittel mit biologischen Materialien, insbesondere Gelatine, Kollagen und/oder Albumin, verschweißt sein.

Das Implantat bzw. das Band kann resorbierbar, teilweise resorbierbar oder nicht resorbierbar ausgebildet sein. Ist das Band resorbierbar bzw. teilweise resorbierbar ausgebildet, so verbleibt nach Resorption bzw. Teilresorption kein bzw. weniger Fremdmaterial im Körper eines Patienten, wodurch das Risiko von Abstoßungsreaktionen minimiert wird.

In einer weitergehenden Ausführungsform ist zumindest der Mittelabschnitt des Bandes resorbierbar ausgebildet. Bevorzugt ist der Mittelabschnitt resorbierbar und zumindest einer der beiden Endabschnitte, vorzugsweise beide Endabschnitte, nicht resorbierbar ausgebildet. Durch die Resorption des Mittelabschnittes kann es zu entzündlichen Reaktionen kommen, die die Neubildung von Bindegewebe, insbesondere in Form von Narbengewebe, begünstigen. Wie bereits erwähnt, ist der Mittelabschnitt des Bandes in der Regel zur Unterstützung bzw. Anhebung von anatomischen Strukturen, insbesondere des Blasenhalses und/oder der Urethra, bevorzugt der bulbären Urethra, vorgesehen. Diese unterstützende bzw. anhebende Funktion des Mittelabschnittes kann somit in besonders vorteilhafter Weise durch das gebildete Bindegewebe bzw. Narbengewebe übernommen werden. Bezüglich geeigneter resorbierbarer bzw. nicht resorbierbarer Materialien für die einzelnen Bandabschnitte wird auf die im Folgenden noch genannten Materialien Bezug genommen.

In einer alternativen weitergehenden Ausführungsform ist zumindest der Mittelabschnitt des Bandes nicht resorbierbar ausgebildet. Bevorzugt ist der Mittelabschnitt nicht resorbierbar und zumindest einer der beiden Endabschnitte, vorzugsweise beide Endabschnitte, resorbierbar ausgebildet. Bezüglich geeigneter resorbierbarer bzw. nicht resorbierbarer Materialien für die einzelnen Bandabschnitte wird ebenfalls auf die im Folgenden noch genannten Materialien verwiesen.

Das Band, insbesondere der Mittelabschnitt und/oder zumindest einer der beiden Endabschnitte, vorzugsweise beide Endabschnitte, sind in einer weiteren Ausführungsform einlagig, zweilagig, dreilagig oder mehrlagig aufgebaut.

Zumindest der Mittelabschnitt, insbesondere nur der Mittelabschnitt, ist in einer weitergehenden Ausführungsform zweilagig aufgebaut, wobei eine Lage aus einem resorbierbarem Material und die andere Lage aus einem nicht resorbierbaren Material gebildet ist. Auf diese Weise kann die unterstützende bzw. anhebende Funktion der nicht resorbierbaren Mittelabschnittslage durch neu gebildetes Bindegewebe, insbesondere Narbengewebe, welches infolge der Resorption der resorbierbaren Mittelabschnittslage entstanden ist, zusätzlich verstärkt werden. Die resorbierbare Lage ist vorzugsweise folienartig ausgebildet, um Gewebeerosionen zu vermeiden. Bezüglich geeigneter resorbierbarer bzw. nicht resorbierbarer Materialien für die einzelnen Lagen wird ebenso auf die im Folgenden noch genannten Materialien verwiesen.

Weiterhin kann es vorgesehen sein, dass das zumindest eine textil ausgebildete Befestigungsmittel, insbesondere nur das zumindest eine textil ausgebildete Befestigungsmittel, resorbierbar ausgebildet ist. Wie eingangs erwähnt, dient das zumindest eine textil ausgebildete Befestigungsmittel hauptsächlich der Befestigung des Bandes an ein geeignetes chirurgisches Einführinstrument, in der Regel an eine chirurgische Nadel. Nach erfolgreicher Implantation des Bandes hat das zumindest eine textil ausgebildete Befestigungsmittel daher seinen hauptsächlichen Zweck erfüllt und ist im Prinzip nicht länger von Nöten. Mittels der in diesem Absatz beschriebenen Ausführungsform lässt sich daher ebenfalls der Anteil an Fremdmaterial im Körper eines Patienten reduzieren.

Das Band weist in einer weiteren vorteilhaften Ausführungsform entlang zumindest eines seiner Längsränder bzw. einer seiner Längskanten, vorzugsweise entlang von beiden Längsrändern bzw. -kanten, für Körperzellen und/oder Körpergewebe hintergreifbare Strukturen auf. Diese Strukturen dienen vorzugsweise einer Primärverankerung des Bandes während und nach der Operation. Die hintergreifbaren Strukturen können aus dem textilen Verbund des Bandes herausragen. Bevorzugt sind die hintergreifbaren Strukturen fest in die textile Struktur des Bandes eingebunden. Um eine dauerhafte Gewebereizung zu vermeiden, kann es gegebenenfalls vorgesehen sein, dass die für Körperzellen und/oder Körpergewebe hintergreifbaren Strukturen resorbierbar ausgebildet sind. Die hintergreifbaren Strukturen können beispielsweise aus der Gruppe bestehend aus Flore, Fransen, texturierte Fäden, Veloursschlingen, Flottungen und Kombinationen davon ausgewählt sein. Bevorzugt handelt es sich bei den für Körperzellen und/oder Körpergewebe hintergreifbaren Strukturen um schlaufen- bzw. schlingenförmige Längskantenfäden. Insoweit wird auch auf die EP 1 361 834 B1 Bezug genommen, deren Offenbarungsgehalt bezüglich der dort beschriebenen Kantenfäden durch ausdrückliche Bezugnahme zum Inhalt der vorliegenden Beschreibung gemacht wird.

Nach einer weiteren Ausführungsform weist das zumindest eine textil ausgebildete Befestigungsmittel für Körperzellen und/oder Körpergewebe hintergreifbare Strukturen auf. Bezüglich möglicher Ausbildungen der hintergreifbaren Strukturen wird auf den vorherigen Absatz verwiesen.

In einer weitergehenden Ausführungsform weist das Band, insbesondere der Mittelabschnitt und/oder zumindest einer der beiden Endabschnitte, vorzugsweise beide Endabschnitte, Fäden mit einer profilierten Oberfläche auf. Bevorzugt weist das Band poröse Fäden auf. Derartige Fäden lassen sich beispielsweise mittels Auslaugungstechniken, sogenannten Leaching-Techniken, herstellen. Beispielsweise können einer Polymerschmelze Salze hinzugegeben werden, ehe die Schmelze zu Fäden ausgesponnen oder extrudiert wird. Nach Abkühlung und Verfestigung der ausgesponnenen bzw. extrudierten Fäden können die Salze dann mittels eines geeigneten Lösungsmittels aus den Fäden herausgelöst werden. Die Verwendung von porösen Fäden zur Herstellung des Bandes bietet unter anderem den Vorteil einer einfacheren Wirkstoffbeladung des Bandes. Grundsätzlich geeignete Wirkstoffe werden im Folgenden noch näher aufgeführt.

Grundsätzlich kann das Band, insbesondere der Mittelabschnitt und/oder zumindest einer der beiden Endabschnitte, vorzugsweise beide Endabschnitte, aus allen biokompatiblen Materialien, vorzugsweise fadenbildenden biokompatiblen Materialien, gebildet sein. Unter einem biokompatiblen Material soll im Sinne der vorliegenden Erfindung ein körper- bzw. gewebeverträgliches Material verstanden werden. Der Mittelabschnitt und die beiden Endabschnitte können dabei aus dem gleichen Material oder aus verschiedenen Materialien gebildet sein. Insbesondere können die beiden Endabschnitte aus dem gleichen Material und der Mittelabschnitt aus einem anderen Material gebildet sein. Des Weiteren kann das Material resorbierbar, teilresorbierbar oder nicht resorbierbar sein. In der Regel handelt es sich bei geeigneten Materialien um Polymere. Bei den Polymeren kann es sich um synthetische Polymere, Biopolymere (natürlich vorkommende Polymere), von Biopolymeren abgeleitete Polymere oder um Kombinationen davon handeln. Bei den vorstehend erwähnten Biopolymeren kann es sich insbesondere um Proteine, bevorzugt extrazelluläre Proteine, und/oder Polysaccharide, bevorzugt um Mucopolysaccharide, handeln. Als Polymere, welche von Biopolymeren abgeleitet sind, können Cellulosederivate, insbesondere Alkylcellulosen, zumindest teilweise deacetylierte Mucopolysaccharide, Salze davon oder Kombinationen davon in Betracht kommen.

In einer bevorzugten Ausführungsform ist das Band, insbesondere der Mittelabschnitt und/oder zumindest einer der beiden Endabschnitte, vorzugsweise beide Endabschnitte, aus einem nicht resorbierbaren synthetischen Polymer gebildet, welches aus der Gruppe bestehend aus Polyolefine, insbesondere Polyethylen, Polyethylen hoher Dichte (HDPE), Polyethylen niedriger Dichte (LDPE), hochmolekulares Polyethylen (HMWPE), ultra-hochmolekulares Polyethylen (UHMWPE), Polypropylen, Polyvinylidendifluorid (PVDF), Polytetrafluorethylen (PTFE) und/oder Polyhexafluorpropylen, Polyester, insbesondere Polyethylenterephthalat (PET) und/oder Polybutylenterephthalat (PBT), Polyamide, Polyurethane, Polyetherketone, Polyphenylensulfid, Copolymere davon und Kombinationen, insbesondere Blends, davon ausgewählt ist.

In einer weiteren bevorzugten Ausführungsform ist das Band, insbesondere der Mittelabschnitt und/oder zumindest einer der beiden Endabschnitte, vorzugsweise beide Endabschnitte, aus einem Polyhydroxyalkanoat, insbesondere auf Basis von α-, β-, γ- und/oder δ-Hydroxycarbonsäuren, gebildet. Bevorzugte Polyhydroxyalkanoate sind aus der Gruppe bestehend aus Polylaktid, Polyglykolid, Poly-ε-Caprolacton, Polytrimethylencarbonat, Poly-para-Dioxanon, Poly-3-Hydroxybutyrat, Poly-4-Hydroxybutyrat, Copolymere davon und Kombinationen, insbesondere Blends, davon ausgewählt. Ein bevorzugtes Copolymer basiert auf Glykolid und Laktid.

Unter Copolymere im Sinne der vorliegenden Erfindung sollen Polymere verstanden werden, welche zumindest zwei unterschiedliche Monomereinheiten aufweisen. Entsprechend werden von dem Begriff Copolymere nicht nur Copolymere im engeren Sinn (Polymer auf Basis von zwei unterschiedlichen Monomereinheiten) erfasst, sondern auch Terpolymere, Tetrapolymere und dergleichen. Des Weiteren kann es sich bei den Copolymeren um statistische oder segmentierte Copolymere (Blockpolymere) handeln.

In einer weiteren bevorzugten Ausführungsform ist das Band, insbesondere der Mittelabschnitt und/oder zumindest einer der beiden Endabschnitte, vorzugsweise beide Endabschnitte, aus einem Biopolymer oder einem davon abgeleiteten Polymer gebildet, welches ausgewählt ist aus der Gruppe bestehend aus Gelatine, Collagen, Elastin, Retikulin, Fibronektin, Albumin, Methylcellulose, Hydroxymethylcellulose, Hydroxyethylcellulose, Hydroxypropylcellulose, Hydroxybutylcellulose, Hydroxyethylmethylcellulose, Hydroxypropylmethylcellulose, Carboxymethylcellulose, Keratansulfat, Dermatansulfat, Chondroitinsulfat, Hyaluronsäure, Chitosan, Stärke, Amylose, Amylopektin, Dextran, Polyvinylalkohol, Salze davon und Kombinationen davon.

In einer weiteren bevorzugten Ausführungsform ist das Band, insbesondere der Mittelabschnitt und/oder zumindest einer der beiden Endabschnitte, vorzugsweise beide Endabschnitte, aus einem biologischen, vorzugsweise tierischen, Gewebe gebildet. Das Gewebe ist vorzugsweise xenogenen, insbesondere porcinen, bovinen oder equinen, Ursprungs. Ein bevorzugtes tierisches Gewebe ist Pericardgewebe, insbesondere bovinen Ursprungs, welches zum Beispiel unter der Bezeichnung Lyoplant^{®} kommerziell erhältlich ist.

In einer weiteren Ausführungsform ist das Band, insbesondere der Mittelabschnitt und/oder zumindest einer der beiden Endabschnitte, vorzugsweise beide Endabschnitte, aus einer Kombination von Materialien gebildet, wie sie insbesondere in den vorhergehenden Ausführungsformen ausführlich beschrieben wurden. Besonders bevorzugt ist ein Band, dessen Mittelabschnitt aus einem resorbierbaren Material, insbesondere einem Polyhydroxyalkanoat wie beispielsweise Polylaktid, Polyglykolid, Polyhydroxybutyrat, Poly-ε-caprolacton, Polytrimethylencarbonat, Poly-p-dioxanon, Copolymere davon oder Kombinationen davon, und dessen beiden Endabschnitte jeweils aus einem Polyolefin, vorzugsweise Polypropylen, gebildet sind. Besonders bevorzugt ist auch ein Band, dessen Mittelabschnitt aus einem Polyolefin, vorzugsweise Polypropylen, und dessen beiden Endabschnitte jeweils aus einem resorbierbaren Material, insbesondere einem Polyhydroxyalkanoat wie beispielsweise Polylaktid, Polyglykolid, Polyhydroxybutyrat, Poly-ε-caprolacton, Polytrimethylencarbonat, Poly-p-dioxanon, Copolymere davon oder Kombinationen davon, gebildet sind. Bei den beiden zuvor beschriebenen Varianten eines erfindungsgemäßen Bandes kann der Mittelabschnitt zusätzlich eine Schutzfolie, vorzugsweise aus einem tierischen Gewebe, beispielsweise aus Pericard, insbesondere bovinen Ursprungs, aufweisen, um Gewebeerosionen zu vermeiden.

Das Band, insbesondere der Mittelabschnitt und/oder zumindest einer der beiden Endabschnitte, vorzugsweise beide Endabschnitte, ist in der Regel aus Fäden gebildet bzw. weist zumindest Fäden auf. Bei den Fäden kann es sich um Monofilamente, Pseudomonofilamente, Multifilamente, Multifilamentgarne und/oder geflochtene Fäden handeln. Bevorzugt ist das Band, insbesondere der Mittelabschnitt und/oder zumindest einer der beiden Endabschnitte, vorzugsweise beide Endabschnitte, aus monofilen Fäden, insbesondere monofilen Polypropylenfäden, gebildet bzw. weist derartige Fäden auf.

Das Implantat bzw. Band, insbesondere der Mittelabschnitt und/oder zumindest einer der beiden. Endabschnitte, vorzugsweise beide Endabschnitte, liegt vorzugsweise in Form eines textilen Netzes vor. Besonders bevorzugt ist das Band als Gewirk, insbesondere Kettengewirk, ausgeführt. Erfindungsgemäß ist es weiterhin bevorzugt, wenn das Band einstückig gefertigt, insbesondere einstückig gewirkt, ist. Beispielsweise kann das Band in einstückiger Form aus einer Netzbahn hergestellt, insbesondere herausgeschnitten oder -gestanzt, sein. Grundsätzlich kann es aber auch vorgesehen sein, dass der Mittelabschnitt und die beiden Endabschnitte separat hergestellt und anschließend miteinander zu dem erfindungsgemäßen Band verbunden werden. Hierzu können die einzelnen Bandabschnitte miteinander verklebt, verschweißt, insbesondere ultraverschweißt, und/oder vernäht sein. Zur Verklebung der Bandabschnitte miteinander können Gewebekleber, beispielsweise auf Cyanoacrylatbasis, verwendet werden. Weitere geeignete Verbindungstechniken können auf Veloursverschlüssen, Klettverschlüssen, Knopflochverschlüssen, Druckknopfverschlüssen, Schlitzverschlüssen oder auf Verschlüssen beruhen, welche auf dem sogenannten Mädchenfänger-Prinzip basieren.

In einer weitergehenden Ausführungsform weist das Band, insbesondere der Mittelabschnitt und/oder zumindest einer der beiden Endabschnitte, bevorzugt zumindest der Mittelabschnitt, eine dreidimensionale, vorzugsweise für Körperzellen und/oder Körpergewebe hintergreifbare, Textilstruktur auf. Bei der dreidimensionalen Textilstruktur handelt es sich vorzugsweise um eine Veloursstruktur, insbesondere Doppelveloursstruktur. Im Falle einer Doppelveloursstruktur können unterschiedliche Flor- bzw. Polhöhen, insbesondere auf gegenüberliegenden Seiten des Bandes, vorzugsweise zumindest des Mittelabschnittes, ausgebildet sein. Eine veloursartige Ausbildung des Bandes kann eine zusätzliche Gewebereizung bewirken, welche ebenfalls die Neubildung von Bindegewebe, insbesondere Narbengewebe, nach Implantation des Bandes stimuliert und auf diese Weise ebenso die Anhebung bzw. Stützung von anatomischen Strukturen begünstigt. Weiterhin kann es sich bei der dreidimensionalen Textilstruktur auch um ein Abstandstextil, insbesondere ein Abstandsgewirk, handeln.

Das Band, insbesondere der Mittelabschnitt und/oder zumindest einer der beiden Endabschnitte, vorzugsweise beide Endabschnitte, kann grundsätzlich in allen textilen Bindungen ausgeführt sein. Beispielsweise kann das Band in Trikotbindung, Trikot-Atlasbindung, Samtbindung, Tuchbindung, Frottierbindung oder Kombinationen davon ausgebildet sein. Eine Kombination von Fransenbindung mit Tuchbindung ist erfindungsgemäß besonders bevorzugt.

Nach einer weiteren Ausführungsform ist das Band, insbesondere der Mittelabschnitt und/oder zumindest einer der beiden Endabschnitte, vorzugsweise beide Endabschnitte, anisotrop elastisch ausgebildet. Mit anderen Worten kann das Band eine anisotrope, d.h. richtungsabhängige, Elastizität besitzen. Beispielsweise ist der Mittelabschnitt elastischer ausgebildet als die beiden Endabschnitte des Bandes. Dies hat den Vorteil, dass sich der Mittelabschnitt besser an Veränderungen, insbesondere Füllungszustände, von anatomischen Strukturen, beispielsweise der Harnblase, anpassen kann. Außerdem ermöglicht eine weniger elastische Ausbildung der beiden Endabschnitte eine festere bzw. straffere und damit sichere Aufhängung des Bandes im Körper eines Patienten. Erfindungsgemäß kann es grundsätzlich jedoch auch möglich sein, dass die beiden Endabschnitte jeweils elastischer ausgebildet sind als der Mittelabschnitt.

In einer weitergehenden Ausführungsform ist das Band in Querrichtung des Mittelabschnittes elastischer ausgebildet als in Längsrichtung des Mittelabschnittes. Insbesondere kann das Band in Querrichtung des Mittelabschnittes elastischer ausgebildet sein als in Längs- und/oder Querrichtung, vorzugsweise Längs- und Querrichtung, von zumindest einem der beiden Endabschnitte, vorzugsweise von beiden Endabschnitten. Die Endabschnitte können im Verhältnis zueinander unterschiedliche Elastizitäten aufweisen. Erfindungsgemäß kann es jedoch ebenso vorgesehen sein, dass das Band im Bereich der beiden Endabschnitte im Wesentlichen isotrop-elastisch ausgebildet ist. Abhängig von den anatomischen Gegebenheiten der Implantationsstelle kann eine der vorstehend beschriebenen Ausführungsformen eines anisotrop-elastisch ausgebildeten Bandes bevorzugt sein. Grundsätzlich kann das Band in toto aber auch isotrop-elastisch ausgebildet sein.

In einer weiteren Ausführungsform ist das Implantat bzw. Band, insbesondere der Mittelabschnitt und/oder zumindest einer der beiden Endabschnitte, vorzugsweise beide Endabschnitte, aus einem leichtgewichtigen Netz gebildet. Bevorzugt besitzt das Band ein Flächengewicht zwischen 25 und 70 g/m², insbesondere 30 und 50 g/m², vorzugsweise 30 und 40 g/m². Ein leichtgewichtiges Band hat den Vorteil, dass sich hierdurch Gewebeerosionen vermeiden, zumindest aber deutlich reduzieren lassen. Erfindungsgemäß kann es daher vorgesehen sein, dass zumindest der Mittelabschnitt des Bandes aus einem leichtgewichtigen Netz, insbesondere wie in diesem Abschnitt beschrieben, gebildet ist.

In einer weitergehenden Ausführungsform ist das Band, insbesondere der Mittelabschnitt und/oder zumindest einer der beiden Endabschnitte, vorzugsweise beide Endabschnitte, aus einem unter der Bezeichnung Optilene^{®} Mesh LP und/oder Optilene^{®} Mesh Elastic von der Anmelderin kommerziell vertriebenen Netz gebildet. Im Falle von Optilene^{®} Mesh LP handelt es sich um ein Netz aus monofilem Polypropylen mit einem Flächengewicht von circa 36 ± 5 g/m², einer Maschengröße von ca. 1 mm und einer Maschendichte von ca. 112 Maschen pro cm². Das Netz Optilene^{®} Mesh Elastic ist ein Netz aus monofilem Polypropylen mit einem Flächengewicht von ca. 48 ± 7 g/m², einer Maschengröße 3,6 x 2,8 mm und einer Maschendichte von ca. 60 Maschen pro cm². Erfindungsgemäß kann es weiterhin vorgesehen sein, dass das Band, insbesondere der Mittelabschnitt und/oder zumindest einer der beiden Endabschnitte, vorzugsweise beide Endabschnitte, eine Netzstruktur besitzt, die der Netzstruktur von Optilene^{®} Mesh LP und/oder Optilene^{®} Mesh Elastic entspricht, wobei die Netzstruktur aus einem anderen Material als Polypropylen, insbesondere aus einem resorbierbaren und/oder nicht resorbierbaren Material, gebildet ist. Bezüglich geeigneter Materialien wird vollumfänglich auf die vorliegende Beschreibung Bezug genommen.

In einer besonders bevorzugten Ausführungsform liegt das Band in Form eines sogenannten Gradientennetzes vor. Unter einem Gradientennetz im Sinne der vorliegenden Erfindung soll ein textiles Verbundnetz aus zumindest zwei, insbesondere zwei oder drei, unterschiedlichen Einzelnetzen verstanden werden, wobei die Einzelnetze flächig nebeneinander angeordnet und miteinander verbunden sind. Bezüglich geeigneter Verbindungstechniken wird auf die in der vorliegenden Beschreibung bereits an anderer Stelle genannten Verbindungstechniken verwiesen. Mit anderen Worten handelt es sich bei einem Gradientennetz um ein bezüglich seiner textilen Eigenschaften, beispielsweise Fadenmaterial, Fadendurchmesser, Fadentiter, Maschen- bzw. Porengröße, Maschen- bzw. Porendichte und dergleichen, polyfunktionales Netz. Beispielsweise kann der Mittelabschnitt des Bandes eine andere textile Netzstruktur bzw. einen anderen textilen Aufbau und damit andere textile Eigenschaften besitzen als die beiden Endabschnitte des Bandes. Die beiden Endabschnitte können die gleiche textile Netzstruktur besitzen. Erfindungsgemäß ist es aber ebenso möglich, dass die beiden Endabschnitte im Verhältnis zueinander unschiedliche Netzstrukturen besitzen. Mit anderen Worten kann es erfindungsgemäß vorgesehen sein, dass jeder Abschnitt (Mittelabschnitt und die beiden Endabschnitte beidseits des Mittelabschnittes) des Bandes eine andere Netzstruktur besitzt.

In einer weiteren Ausführungsform weist das Band, insbesondere der Mittelabschnitt und/oder zumindest einer der beiden Endabschnitte, vorzugsweise die beiden Endabschnitte, auf zumindest einer Flächenseite davon, eine Beschichtung oder Imprägnierung auf. Die Beschichtung bzw. Imprägnierung ist vorzugsweise glatt und insbesondere abdichtend ausgebildet. Bevorzugt ist die Beschichtung bzw. Imprägnierung als Folie oder Film ausgebildet. Die Beschichtung bzw. Imprägnierung ist zweckmäßigerweise aus einem bioverträglichen Material gebildet. Das Material ist bevorzugt resorbierbar, zumindest aber teilresorbierbar, ausgebildet. Grundsätzlich kann es sich bei dem Material um ein resorbierbares synthetisches Polymer bzw. Copolymer, insbesondere um ein Polyhydroxyalkanoat bzw. Polyhydroxyalkanoatcopolymer, handeln. Insoweit wird vollumfänglich auf die bisherige Beschreibung Bezug genommen. Bevorzugt ist das Material ein Biopolymer, vorzugsweise ein Protein, ein Polysaccharid, ein Salz davon oder eine Kombination davon. Als Proteine können extrazelluläre Proteine bzw. deren Salze in Betracht kommen. Bevorzugte Polysaccharide sind Mucopolysaccharide, zumindest teilweise deacetylierte Mucopolysaccharide, Cellulosederivate, insbesondere Alkylcellulosen, Salze davon oder Kombinationen davon. Bevorzugt ist das Material der Beschichtung bzw. Imprägnierung aus der Gruppe bestehend aus Gelatine, Collagen, Retikulin, Elastin, Fibronektin, Albumin, Methylcellulose, Hydroxymethylcellulose, Hydroxyethylcellulose, Hydroxypropylcellulose, Hydroxybutylcellulose, Hydroxyethylmethylcellulose, Hydroxypropylmethylcellulose, Carboxymethylcellulose, Chitosan, Hyaluronsäure, Heparansulfat, Dermatansulfat, Keratansulfat, Chondroitinsulfat, Stärke, Chitosan, Dextran, Amylopektin, Amylose, Polyvinylalkohol, Salze davon und Kombinationen davon ausgewählt. Als biologisches Gewebe kommt vorzugsweise tierisches Gewebe, bevorzugt xenogenen, insbesondere porcinen, bovinen oder equinen, Ursprungs, in Betracht. Ein bevorzugtes Gewebe stellt Pericardgewebe, insbesondere bovinen Urpsrungs, dar. Beispielsweise kann es sich bei dem biologischen Gewebe um ein kommerziell unter der Bezeichnung Lyoplant^{®} erhältliches Pericard handeln. Mittels einer Beschichtung, wie sie in diesem Absatz beschrieben ist, können beispielsweise unerwünschte Gewebeadhäsionen mit anatomischen Strukturen, insbesondere der Harnblase, dem Harnblasenhals und/oder der Harnröhre, insbesondere der bulbären Harnröhre, und/oder unerwünschte Gewebeerosionen vermieden werden.

In einer weitergehenden Ausführungsform weist nur der Mittelabschnitt eine Beschichtung oder Imprägnierung auf. Die Beschichtung bzw. Imprägnierung kann insbesondere als Schutzfolie oder Schutzfilm zur Vermeidung von Gewebeerosionen ausgebildet sein. Bezüglich weiterer Einzelheiten und Merkmale zu der Beschichtung bzw. Imprägnierung wird vollumfänglich auf die im vorherigen Absatz gemachten Ausführungen verwiesen.

Das Band, insbesondere der Mittelabschnitt und/oder zumindest einer der beiden Endabschnitte, vorzugsweise die beiden Endabschnitte, kann weiterhin Additive, insbesondere biologische, medizinische und/oder pharmazeutische Wirkstoffe und/oder detektierbare Zusätze, aufweisen. Geeignete Additive können beispielsweise aus der Gruppe bestehend aus antimikrobielle, insbesondere antibiotische, Wirkstoffe, desinfizierende Wirkstoffe, entzündungshemmende Wirkstoffe, schmerzlindernde Wirkstoffe, zelluläre Wachstumsfaktoren, zelluläre Rekrutierungsfaktoren, zelluläre Differenzierungsfaktoren, zelluläre Adhäsionsfaktoren, Röntgenkontrastmittel, insbesondere Bariumsulfat, und Kombinationen davon ausgewählt sein. Unter antimikrobiellen Wirkstoffen sollen im Sinne der vorliegenden Erfindung Wirkstoffe verstanden werden, welche allgemein die Vermehrungsfähigkeit und/oder Infektiosität von Mikroorganismen reduzieren und/oder sie abtöten. Als antimikrobielle Wirkstoffe sind in erster Linie Metalle, Metalllegierungen und/oder Metallverbindungen, insbesondere Metallsalze, zu nennen. Bevorzugte antimikrobielle Wirkstoffe sind demnach aus der Gruppe bestehend aus metallisches Kupfer, Zink, Silber, Gold, Platin, Titan, Kombinationen davon und Verbindungen, insbesondere Legierungen und/oder Salze, davon ausgewählt. Weitere Beispiele für antimikrobielle Wirkstoffe können aus der Gruppe bestehend aus Chitosan, Poly-ε-Lysin, Polyhexamethylenbiguanid, Akazid^{®}, Cyclohexidin, Cetyltrimethylammoniumbromid (CTAB), Octenidin, Benzalkoniumchlorid, Cetylpyridiniumchlorid, Salze davon, insbesondere Fettsalze davon, und Kombinationen davon ausgewählt sein.

In einer weiterführenden Ausführungsform liegen die im vorherigen Abschnitt beschriebenen Additive in partikulärer Form, insbesondere als Mikro- und/oder Nanopartikel, vor.

Das Band gemäß der vorliegenden Erfindung liegt in der Regel in sterilisierter und insbesondere konfektionierter Form vor.

Wie bereits mehrfach erwähnt, eignet sich das erfindungsgemäße Implantat vor allem zur Behandlung der Harninkontinenz. Mit anderen Worten handelt es sich bei dem Implantat vorzugsweise um ein Harninkontinenzband. Als solches kann es zur Behandlung der männlichen und/oder weiblichen Harninkontinenz verwendet werden. Erfindungsgemäß kann es weiterhin vorgesehen sein, dass das Implantat zur Beckenbodenrekonstruktion, insbesondere zur Versorgung oder Behandlung von Prolapsen oder prolapsartigen Erkrankungen, oder zur Vermeidung von Dyspareunia verwendet wird.

Beschrieben ist auch ein chirurgisches Implantat, insbesondere zur Anwendung bei der Behandlung von Harninkontinenz, mit einer Schlauch- oder Röhrenstruktur und einem textilen Band innerhalb der Schlauch- oder Röhrenstruktur.

Das textile Band ist vorzugsweise zur Anhebung bzw. Unterstützung von anatomischen Strukturen, insbesondere eines Blasenhalses und/oder einer Harnröhre, vorzugsweise einer bulbären Harnröhre, vorgesehen. Die Schlauch- bzw. Röhrenstruktur, die vorzugsweise an beiden Enden über den Querschnitt offen ausgebildet ist, dient in besonders vorteilhafter Weise als Hinterlegungsstruktur für das textile Band und ermöglicht eine korrekte Platzierung des Bandes im Körper eines Patienten, ohne dass es zu einer Beeinträchtigung des Bandes und seiner Eigenschaften im Zuge der Implantation kommt.

In einer bevorzugten Ausführungsform ist die Schlauch- bzw. Röhrenstruktur porös, insbesondere großporig, ausgebildet.

In einer besonders bevorzugten Ausführungsform besitzt die Schlauch- bzw. Röhrenstruktur eine Textilstruktur, insbesondere eine Flecht- oder Wirkstruktur, vorzugsweise Flechtstruktur. Mit anderen Worten handelt es sich bei der Schlauch- bzw. Röhrenstruktur vorzugsweise um ein Schlauch- bzw. Röhrengeflecht (Rundgeflecht).

Die Schlauch- bzw. Röhrenstruktur kann grundsätzlich aus einem resorbierbaren oder nicht resorbierbaren Material gebildet sein. Bevorzugt die ist Schlauch- bzw. Röhrenstruktur jedoch aus einem resorbierbaren Material gebildet. Das Material kann eine in vivo Resorptionsdauer zwischen 40 und 300 Tagen aufweisen. Bezüglich geeigneter resorbierbarer bzw. nicht resorbierbarer Materialien wird vollumfänglich auf die in der bisherigen Beschreibung genannten Materialien Bezug genommen. Besonders erwähnt werden sollen an dieser Stelle jedoch noch einmal Polyhydroxyalkanoate als Beispiele für resorbierbare Materialien, insbesondere ausgewählt aus der Gruppe bestehend aus Polylaktid, Polyglykolid, Poly-ε-Caprolacton, Polytrimethylencarbonat, Poly-para-Dioxanon, Poly-3-Hydroxybutyrat, Poly-4-Hydroxybutyrat, Copolymere davon und Kombinationen, insbesondere Blends, davon.

Das textile Band befindet sich zweckmäßigerweise möglichst mittig innerhalb der Schlauch- bzw. Röhrenstruktur.

Das textile Band kann grundsätzlich aus einem resorbierbaren oder nicht resorbierbaren Material gebildet sein. Bevorzugt ist das Band aus einem nicht resorbierbaren Material gebildet. Bezüglich weiterer Einzelheiten zu dem Band, insbesondere in Bezug auf dessen textile Eigenschaften und/oder Materialien, aus denen das Band gebildet sein kann, wird vollumfänglich auf die bisherige Beschreibung Bezug genommen. Besonders erwähnt werden sollen an dieser Stelle jedoch noch einmal Polyolefine, insbesondere Polypropylen, Copolymere davon oder Kombinationen, insbesondere Blends, davon als Beispiele für nicht resorbierbare Materialien, aus denen das Band gebildet sein kann.

In einer besonders bevorzugten Ausführungsform ist die Schlauch- bzw. Röhrenstruktur aus einem resorbierbaren Material und das textile Band aus einem nicht resorbierbaren Material gebildet. Diese Ausführungsform besitzt den Vorteil, dass letztlich lediglich das textile Band als Fremdkörper im Körper eines Patienten verbleibt und durch die Resorption der Schlauch- bzw. Röhrenstruktur neues Bindegewebe entsteht, welches das Band in seiner vorzugsweise stützenden Funktion unterstützt.

Zur Befestigung an ein chirurgisches Einführinstrument, insbesondere an eine chirurgische Nadel, kann die Schlauch- bzw. Röhrenstruktur im Bereich ihrer Enden Öffnungen, insbesondere schlitzförmige Öffnungen, aufweisen. Zur Ausbildung der Öffnungen kann die Schlauch- bzw. Röhrenstruktur im Bereich ihrer Enden zumindest teilweise eingeschnitten sein. Zur Gewebefixierung kann die Schlauch- bzw. Röhrenstruktur, beginnend von ihren Enden, teilweise eingeschnitten sein.

Ein weiterer Aspekt der vorliegenden Erfindung betrifft ein Verfahren zur Herstellung eines Implantates, vorzugsweise zur Behandlung von Harninkontinenz, umfassend die folgenden Schritte:
a) Bereitstellen eines Bandes mit textiler Struktur, einem Mittelabschnitt und zwei Endabschnitten beidseits des Mittelabschnittes,
b) Ausbilden zumindest eines textilen Befestigungsmittels im Bereich zumindest eines der beiden Endabschnitte, vorzugsweise im Bereich von beiden Endabschnitten.

Dabei wird zum Ausbilden des zumindest einen textilen Befestigungsmittels während der Herstellung des bereitzustellenden Bandes im Bereich zumindest eines der beiden Endabschnitte, vorzugsweise im Bereich von beiden Endabschnitten, zumindest ein Faden aus der sich bildenden Textilstruktur herausgeführt und anschließend unter Ausbildung zumindest eines vorzugsweise schlaufen- oder schlingenförmigen textilen Befestigungsmittels wieder in die Textilstruktur des Bandes eingeführt. Alternativ oder in Kombination dazu kann zum Ausbilden des zumindest einen textilen Befestigungsmittels zumindest ein zusätzlicher Faden, gegebenenfalls mehrere zusätzliche Fäden, in die textile Bandstruktur des bereitzustellenden Bandes eingeführt werden.

Zur Adjustierung der Spannung des Mittelabschnittes beim Implantieren des Bandes ist es bevorzugt, wenn zumindest einer der beiden Endabschnitte, vorzugsweise beide Endabschnitte, mit einer Öffnung, vorzugsweise einer schlitzförmigen Öffnung, versehen wird. Die Öffnung kann beispielsweise mittels Laserschneiden oder Ultraschallschneiden erzeugt werden. Weiterhin kann die Öffnung durch mechanisches Einschneiden des Bandes im Bereich zumindest eines der beiden Endabschnitte, vorzugsweise im Bereich von beiden Endabschnitten, erzeugt werden, beispielsweise mit Hilfe eines Messers oder Mikrotoms. Des Weiteren kann die Öffnung auch durch Ausstanzen gebildet werden.

In einer alternativen Ausführungsform wird ein Band bereitgestellt, welches zumindest im Bereich eines der beiden Endabschnitte, vorzugsweise im Bereich von beiden Endabschnitten, zumindest einen, insbesondere einen, in Längsrichtung des Bandes verlaufenden Trenn- bzw. Separierfaden aufweist. Durch Entfernen, insbesondere Herausschneiden, des zumindest einen Trenn- bzw. Separierfadens aus der textilen Bandstruktur wird eine vorzugsweise schlitzförmige Öffnung erzeugt, mittels derer sich die Spannung des Mittelabschnittes beim Implantationsvorgang einstellen lässt.

Weiterhin kann es bevorzugt sein, das zumindest eine textile Befestigungsmittel zu verstärken bzw. versteifen. Hierzu kann das zumindest eine textile Befestigungsmittel thermofixiert und/oder mit anderen Materialien, insbesondere biologischen Materialien wie beispielsweise Gelatine, Kollagen und/oder Albumin, verschweißt, insbesondere ultraschallverschweißt, werden. Eine weitere Möglichkeit besteht darin, das zumindest eine textile Befestigungsmittel textil zu verstärken bzw. zu versteifen. Beispielsweise können mehrere textile Befestigungsmittel verzwirbelt werden, beispielsweise zu einer Kordel.

Schließlich betrifft die vorliegende Erfindung auch ein chirurgisches Set bzw. Kit, umfassend ein Implantat bzw. Band gemäß der vorliegenden Erfindung und zumindest ein chirurgisches Einführinstrument, vorzugsweise zumindest eine chirurgische Nadel. In der Regel umfasst das chirurgische Set ein oder zwei chirurgische Einführinstrumente, insbesondere chirurgische Nadeln. Bezüglich weiterer Merkmale, Einzelheiten und Vorteile wird vollständig auf die bisherige Beschreibung Bezug genommen.

Weitere Einzelheiten und Merkmale sowie Vorteile der Erfindung ergeben sich aus der nachfolgenden Beschreibung anhand der Figuren, der dazugehörigen Figurenbeschreibungen in Kombination mit den Unteransprüchen. Hierbei können einzelne Merkmale der Erfindung alleine oder in Kombination mit anderen Merkmalen verwirklicht sein. Die Figurenbeschreibungen sind dabei lediglich als eine beschreibende, keineswegs als in irgendeiner Weise limitierende Offenbarung zu verstehen. Die Figuren werden hiermit durch ausdrückliche Bezugsnahme zum Inhalt dieser Beschreibung gemacht.

In den Figuren zeigen:
- Figur 1:: eine Ausführungsform eines erfindungsgemäßen Implantates,
- Figuren 2a, b:: weitere Ausführungsformen eines erfindungsgemäßen Implantates,
- Figur 3:: Ausschnitt aus einem Endlosband zur Herstellung von erfindungsgemäßen Implantaten,
- Figur 4:: ein Implantat mit Schlauch- bzw. Röhrenstruktur und einem textilen Band innerhalb der Schlauch- bzw. Röhrenstruktur.

### Figurenbeschreibung

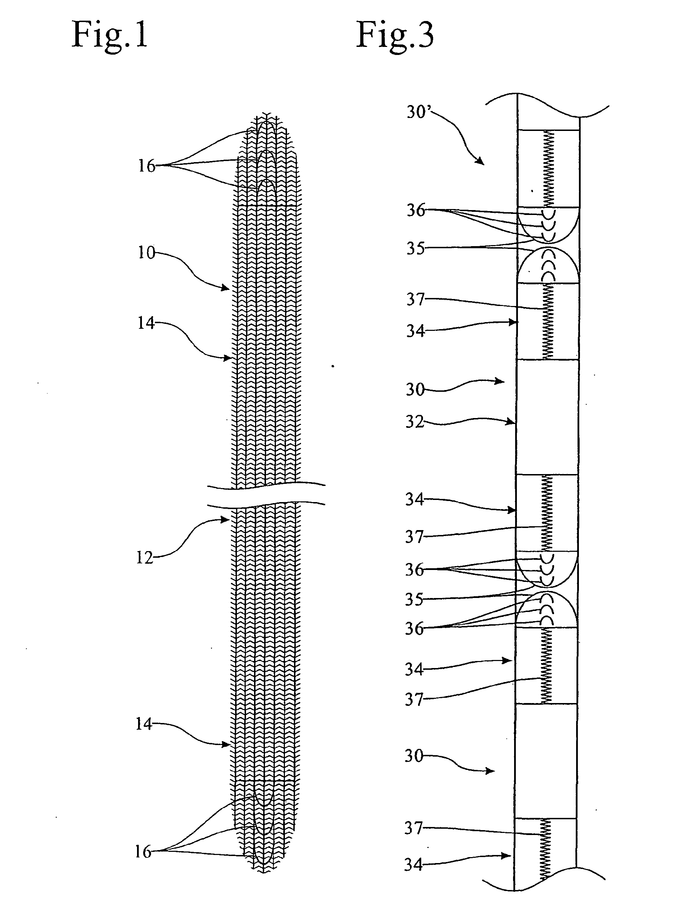

Figur 1 zeigt schematisch ein erfindungsgemäßes Implantat in Form eines Bandes 10, welches bevorzugt zur Behandlung von Harninkontinenz verwendet wird. Das Band 10 besitzt eine textile Struktur und umfasst einen Mittelabschnitt 12 sowie zwei Endabschnitte 14 beidseits des Mittelabschnittes 12. Die Endabschnitte 14 besitzen jeweils ein sich im Durchmesser verjüngendes und abgerundetes Ende.

Bei der textilen Bandstruktur handelt es sich vorzugsweise um eine Netzstruktur. Besonders bevorzugt ist das Band 10 als gewirktes Netz ausgebildet. Das Band 10 kann aus Polypropylenfäden aufgebaut sein. Das Band 10 kann außerdem eine einheitliche Netzstruktur besitzen. Mit anderen Worten können Mittelabschnitt 12 und die beiden Endabschnitte 14 die gleiche Netzstruktur aufweisen. Insbesondere kann das Band 10 in einstückiger Form aus einer Netzbahn hergestellt sein.

Alternativ können Mittelabschnitt 12 und/oder die Endabschnitte 14 auch unterschiedliche Netzstrukturen besitzen und als sogenanntes Gradienten-Netz vorliegen. Hierzu kann das Band 10 in einstückiger Form aus einer entsprechenden Netzbahn hergestellt sein. Die einzelnen Abschnitte 12; 14 des Bandes 10 können sich in Bezug auf Elastizitätsverhalten,- Maschen- bzw. Porenweiten, Fadenmaterial, Fadendurchmesser, Fadentiter, Reißkraftverhalten und dergleichen unterscheiden. Beispielsweise kann es erfindungsgemäß vorgesehen sein, dass der Mittelabschnitt 12 aus einem Netz, welches kommerziell unter der Bezeichnung Opitlene^{®} Mesh LP vertrieben wird, hergestellt ist. Die beiden Endabschnitte 14, welche den Mittelabschnitt 12 flankieren, können beispielsweise aus dem unter der Bezeichnung Optilene^{®} Mesh Elastic kommerziell vertriebenen Netz gebildet sein. Beide Netztypen wurden in der vorhergehenden Beschreibung eingehend charakterisiert. Das Band 10 ist bevorzugt mit Silber und/oder einer Silberverbindung, insbesondere einem Silbersalz, antimikrobiell ausgerüstet.

Zur Befestigung an ein chirurgisches Einführinstrument, in der Regel an eine chirurgische Nadel, besitzt das in Figur 1 dargestellte Implantat im Bereich seiner beiden Endabschnitte 14 jeweils zumindest ein textil ausgebildetes Befestigungsmittel, beispielsweise - wie in Figur 1 dargestellt - jeweils drei als Fadenschlingen (loop) 16 ausgebildete textile Befestigungsmittel. Die Fadenschlingen 16 können von originären Bandfäden und/oder zusätzlich in die Bandstruktur eingeführten Fäden gebildet sein. Die Fadenschlingen 16 sind vorzugsweise als Veloursschlingen oder Flottungen ausgebildet. Mittels der Fadenschlingen 16 ist eine für den Chirurgen komplikationslose Befestigung des Bandes 10 an ein chirurgisches Einführinstrument, beispielsweise im Handgriff- oder Schaftbereich einer chirurgischen Nadel, möglich. Durch die Zugkraft, welche der Operateur über das chirurgische Einführinstrument auf das Band 10, nachdem dieses mit dem Einführinstrument verbunden ist, ausübt, kommt es vorzugsweise zu einer Elongation unter gleichzeitiger Durchmesserverjüngung des Bandes 10. Dies ermöglicht in besonders vorteilhafter Weise einen glatten und insbesondere nahezu atraumatischen Gewebeeinzug des Bandes 10.

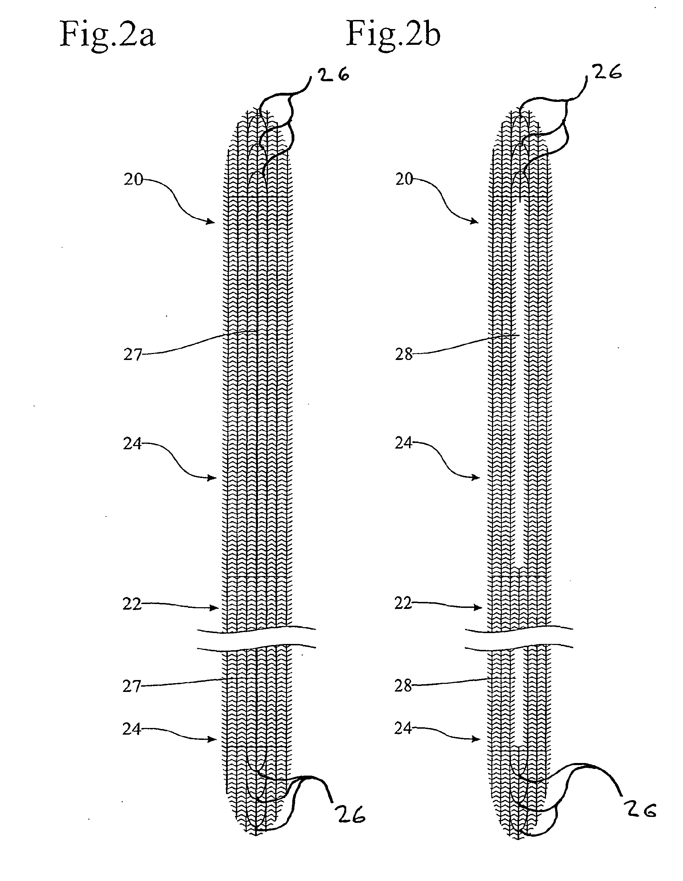

Das in Figur 2a dargestellte Implantat 20 besitzt im Bereich seiner beiden Endabschnitte 24 jeweils einen sich in Längsrichtung des Bandes 20 bzw. von dessen Endabschnitten 24 erstreckenden Trenn- bzw. Separierfaden 27. Vor Implantation des Bandes 20 können die Trenn- bzw. Separierfäden 27 jeweils aus der Bandstruktur entfernt werden, wobei vorzugsweise schlitzförmige Öffnungen 28 entstehen (vgl. hierzu das in der Figur 2b dargestellte Implantat). Die vorzugsweise schlitzförmigen Öffnungen 28 erlauben in besonders vorteilhafter Weise eine Einstellung der Spannung des Mittelabschnittes 22 während der Implantation des Bandes 20, d.h. ermöglichen Zug- und/oder Entlastungsbewegungen des Mittelabschnittes 22. Die Trennfäden 27 bzw. die vorzugsweise schlitzförmigen Öffnungen 28 können jeweils im Bereich ihrer Enden mittels einer in Querrichtung des Bandes 20 verlaufenden Naht 29 abgesichert sein. Hierdurch kann insbesondere ein (weiteres) Aufreißen der vorzugsweise schlitzförmigen Öffnungen 28 vermieden werden. Jeder Endabschnitt 24 weist zumindest ein textiles Befestigungsmittel, beispielsweise in Form von drei Fadenschlingen 26 (wie in den Figuren 2a und 2b dargestellt), auf. Bezüglich weiterer Merkmale, Einzelheiten und Vorteile zu den in Figur 2 dargestellten Implantaten 20 wird vollständig auf die Figurenbeschreibung zur Figur 1 Bezug genommen.

In der Figur 3 ist schematisch ein Ausschnitt aus einem Endlosband 30' wiedergegeben, aus welchem erfindungsgemäße Implantate 30 hergestellt werden können. Jedes Implantat 30 basiert jeweils auf einem Mittelabschnitt 32 sowie zwei Endabschnitten 34 beidseits des Mittelabschnittes 32. Die Implantate 30 können beispielsweise mittels eines Lasers entlang der gekrümmten Linien 35 aus dem Endlosband 30' herausgeschnitten werden. Zur besseren Übersetzung einer Zugbeanspruchung der Endabschnitte 34 auf den Mittelabschnitt 32 können die Endabschnitte 34 entlang der gezackten Linien 37 unter Durchbrechung des Bandmaterials eingeschnitten werden. Dies kann beispielsweise ebenfalls mit Hilfe eines Lasers geschehen. Zur Befestigung an ein chirurgisches Einführinstrument weisen die Implantate 30 jeweils im Bereich ihrer Endabschnitte 34 textile Befestigungsmittel in Form von Fadenschlingen 36 auf. Bezüglich weiterer Merkmale und Vorteile wird auf die vorangegangene Beschreibung, insbesondere auf die Figurenbeschreibungen zu den Figuren 1 und 2, Bezug genommen.

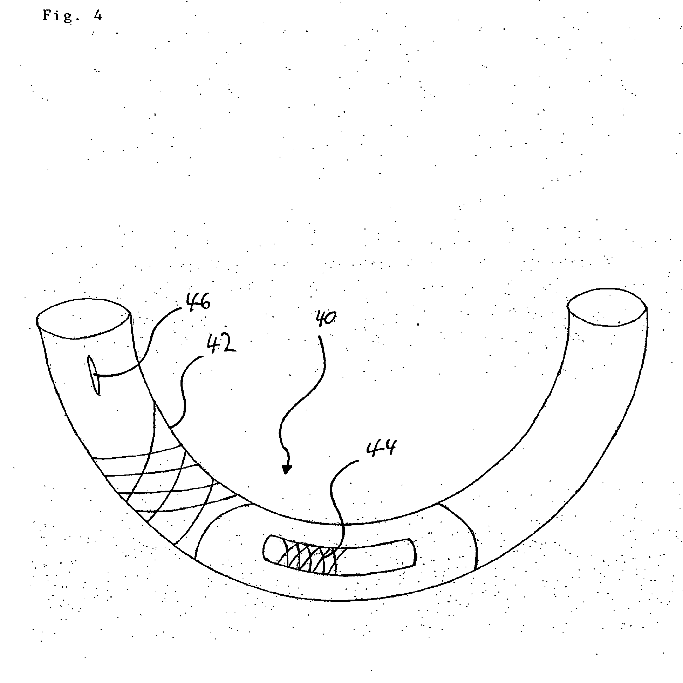

In der Figur 4 ist schematisch ein Implantat 40 dargestellt, dass auf einer Schlauch- bzw. Röhrenstruktur 42 und einem textilen Band 44 basiert, das sich innerhalb der Schlauch- bzw. Röhrenstruktur 42 befindet. Die Schlauch- bzw. Röhrenstruktur 42 ist vorzugsweise als resorbierbares Schlauch- bzw. Röhrengeflecht ausgebildet. Die Schlauch- bzw. Röhrenstruktur 42 ist vorzugsweise aus Polyhydroxyalkanoaten bzw. Copolymeren davon gebildet. Das textile Band 44, welches insbesondere als Gewirk ausgeführt sein kann, ist vorzugsweise aus einem nicht resorbierbaren Material, insbesondere aus einem Polyolefin oder einem Polyolefincopolymer, beispielsweise Polypropylen, gebildet. Die Schlauch- bzw. Röhrenstruktur 42 dient mit besonderem Vorteil als Hinterlegungsstruktur für das textile Band 44 und ermöglicht insbesondere eine störungsfreie Implantation des Bandes 44 in den Körper eines Patienten. Zur Befestigung an ein chirurgisches Einführinstrument, in der Regel an eine chirurgische Nadel, weist die Schlauch- bzw. Röhrenstruktur 42 im Bereich zumindest einer ihrer Enden eine Öffnung 46 auf, mittels derer das Implantat 40 an einem Einführinstrument befestigt werden kann, beispielsweise über einen einfachen Aufhängmechanismus. Ist die Schlauch- bzw. Röhrenstruktur 42 resorbierbar und das Band 44 nicht resorbierbar ausgebildet, verbleibt letztlich nur das Band 44 als Fremdmaterial im Körper des Patienten. Insgesamt werden operative Interventionen zur Anhebung bzw. Unterstützung von anatomischen Strukturen bei Verwendung des Implantats 40 deutlich vereinfacht und die Lebensqualität des Patienten deutlich verbessert. Die Ausführungsform gemäß Figur 4 ist nicht Gegenstand der vorliegenden Erfindung.

## Patentansprüche

1. Chirurgisches Implantat (10; 20; 30), vorzugsweise zur Anwendung bei der Behandlung von Harninkontinenz, in Form eines Bandes mit textiler Struktur, einem Mittelabschnitt (12; 22; 32) und zwei Endabschnitten (14; 24; 34) beidseits des Mittelabschnitts (12; 22; 32), wobei das Band im Bereich zumindest eines der beiden Endabschnitte (14; 24; 34) zumindest ein textil ausgebildetes Befestigungsmittel zur Befestigung des Bandes an ein chirurgisches Einführinstrument, vorzugsweise an eine chirurgische Nadel, aufweist, **dadurch gekennzeichnet, dass** das zumindest eine textil ausgebildete Befestigungsmittel zumindest einen Faden aufweist, der am Aufbau der textilen Struktur des Bandes beteiligt ist.

2. Chirurgisches Implantat (10; 20; 30) nach Anspruch 1, **dadurch gekennzeichnet, dass** das Band im Bereich von beiden Endabschnitten (14; 24; 34) jeweils zumindest ein textil ausgebildetes Befestigungsmittel aufweist.

3. Chirurgisches Implantat (10; 20; 30) nach Anspruch 1 oder 2, **dadurch gekennzeichnet, dass** das zumindest eine textil ausgebildete Befestigungsmittel mehrere Fäden aufweist, die am Aufbau der textilen Struktur des Bandes beteiligt sind.

4. Chirurgisches Implantat (10; 20; 30) nach einem der vorhergehenden Ansprüche, **dadurch gekennzeichnet, dass** das zumindest eine textil ausgebildete Befestigungsmittel als Fadenschlinge (16; 26; 36), insbesondere Veloursschlinge und/oder Flottung, ausgebildet ist.

5. Chirurgisches Implantat (10; 20; 30) nach einem der vorhergehenden Ansprüche, **dadurch gekennzeichnet, dass** das zumindest eine textil ausgebildete Befestigungsmittel zumindest einen zusätzlich in die Bandstruktur eingezogenen Faden, insbesondere mehrere zusätzlich in die Bandstruktur eingezogene Fäden, umfasst.

6. Chirurgisches Implantat (10; 20; 30) nach einem der vorhergehenden Ansprüche, **dadurch gekennzeichnet, dass** das zumindest eine textil ausgebildete Befestigungsmittel als strang- oder schnurförmiges Textilgebilde, insbesondere als Kordel oder Geflecht, insbesondere Rund- oder Schlauchgeflecht, ausgebildet ist.

7. Chirurgisches Implantat (20) nach einem der vorhergehenden Ansprüche, **dadurch gekennzeichnet, dass** zumindest ein Endabschnitt (24), vorzugsweise beide Endabschnitte (24), eine schlitzförmige Öffnung (28) aufweist und die Öffnung (28) vorzugsweise mittels Entfernen zumindest eines in Längsrichtung des zumindest einen Endabschnittes (24) verlaufenden Trenn- bzw. Separierfadens (27) aus der textilen Struktur des Bandes gebildet ist.

8. Chirurgisches Implantat (10; 20; 30) nach einem der vorhergehenden Ansprüche, **dadurch gekennzeichnet, dass** das Band als textiles Netz, insbesondere als textiles Verbundnetz, vorzugsweise als Gewirk, insbesondere Kettengewirk, ausgebildet ist.

9. Chirurgisches Implantat (10; 20; 30) nach einem der vorhergehenden Ansprüche, **dadurch gekennzeichnet, dass** das Band, insbesondere der Mittelabschnitt (12; 22; 32), eine dreidimensionale, vorzugsweise für Körperzellen hintergreifbare, Textilstruktur besitzt, vorzugsweise als Velours, insbesondere Doppelvelours, ausgebildet ist.

10. Chirurgisches Implantat (10; 20; 30) nach einem der vorhergehenden Ansprüche, **dadurch gekennzeichnet, dass** das Band in Trikotbindung, Trikot-Atlasbindung, Samtbindung, Tuchbindung, Frottierbindung oder Kombinationen davon ausgeführt ist.

11. Verfahren zur Herstellung eines chirurgischen Implantats (10; 20; 30) nach einem der vorhergehenden Ansprüche, umfassend die folgenden Schritte:
a) Bereitstellen eines Bandes mit textiler Struktur, einem Mittelabschnitt (12; 22; 32) und zwei Endabschnitten (14; 24; 34) beidseits des Mittelabschnittes (12; 22; 32),
b) Ausbilden zumindest eines textilen Befestigungsmittels im Bereich zumindest eines der beiden Endabschnitte (14; 24; 34),
**dadurch gekennzeichnet, dass** zum Ausbilden des zumindest einen textilen Befestigungsmittels beim Herstellen des Bandes zumindest ein Faden im Bereich des zumindest einen Endabschnittes (14; 24; 34) aus der sich bildenden Textilstruktur herausgeführt und anschließend unter Ausbildung eines vorzugsweise schlaufen- oder schlingenförmigen textilen Befestigungsmittels (16; 26; 36) wieder in die Textilstruktur des Bandes eingeführt wird.

12. Verfahren nach Anspruch 11 **dadurch gekennzeichnet, dass** zum Ausbilden des zumindest einen textilen Befestigungsmittels zumindest ein zusätzlicher Faden, insbesondere mehrere Fäden, in die textile Bandstruktur eingeführt wird.

13. Verfahren nach Anspruch 11 oder 12 **dadurch gekennzeichnet, dass** ein Band bereitgestellt wird, dessen textile Struktur im Bereich zumindest eines der beiden Endabschnitte (24) zumindest einen in Längsrichtung des Bandes verlaufenden Trennfaden (27) aufweist, wobei vorzugsweise der zumindest eine Trennfaden (27) unter Ausbildung einer vorzugsweise schlitzförmigen Öffnung (28) aus der textilen Bandstruktur entfernt wird.

14. Verfahren nach einem der Ansprüche 11 bis 13 **dadurch gekennzeichnet, dass** das zumindest eine textil ausgebildete Befestigungsmittel versteift, insbesondere thermofixiert, ultraschallverschweißt oder textil versteift, insbesondere verzwirbelt, wird.

15. Chirurgisches Set, umfassend ein chirurgisches Implantat nach einem der Ansprüche 1 bis 10 und zumindest ein chirurgisches Einführinstrument, vorzugsweise zumindest eine chirurgische Nadel.

## Claims

1. A surgical implant (10; 20; 30), preferably for application in the treatment of urinary incontinence, in the form of a strip having a textile structure, including a mid-segment (12; 22; 32) and two end segments (14; 24; 34) on both sides of the mid-segment (12; 22; 32), with the strip having at least one textile attachment means in the vicinity of at least one of the two end segments (14; 24; 34) to attach the strip to a surgical insertion device, preferably a surgical needle, **characterized in that** the at least one textile attachment means includes at least one thread involved in the composition of the textile structure of the strip.

2. The surgical implant (10; 20; 30) according to claim 1, **characterized in that** the strip includes at least one textile attachment means in the vicinity of each of the two end segments (14; 24; 34).

3. The surgical implant (10; 20; 30) according to claim 1 or 2, **characterized in that** the at least one textile attachment means includes multiple threads involved in the composition of the textile structure of the strip.

4. The surgical implant (10; 20; 30) according to any of the preceding claims, **characterized in that** the at least one textile attachment means is designed as a thread loop (16; 26; 36), in particular a velour loop and/or a floating thread.

5. The surgical implant (10; 20; 30) according to any of the preceding claims, **characterized in that** the at least one textile attachment means comprises at least one additional thread inserted into the strip structure, particularly multiple additional threads inserted into the strip structure.

6. The surgical implant (10; 20; 30) according to any of the preceding claims, **characterized in that** the at least one textile attachment means is designed as a strand-type or string-type textile material, particularly a cord or a braid, particularly a round braid or a braided hose.

7. The surgical implant (20) according to any of the preceding claims, **characterized in that** at least one end segment (24), preferably both end segments, has/have a slot-like opening (28), and the opening (28) is formed preferably by means of removing at least one parting or separating thread (27) extending in a longitudinal direction of the at least one end segment (24) from the textile structure of the strip.

8. The surgical implant (10; 20; 30) according to any of the preceding claims, **characterized in that** the strip is formed as a textile mesh, particularly a textile composite mesh, preferably a knitted fabric, particularly a warp-knitted fabric.

9. The surgical implant (10; 20; 30) according to any of the preceding claims, **characterized in that** the strip, particularly the mid-segment (12; 22; 32), has a three-dimensional textile structure, preferably adapted to allow engagement of somatic cells therein, preferably formed as velour, particularly double velour.

10. The surgical implant (10; 20; 30) according to any of the preceding claims, **characterized in that** the strip is designed in tricot weave, tricot-satin weave, velvet weave, plane weave, terry weave or any combination thereof.

11. A method for the manufacture of a surgical implant (10; 20; 30) according to any of the preceding claims, comprising the followings steps:
a) providing a strip having a textile structure, including a mid-segment (12; 22; 32) and two end segments (14; 24; 34) on both sides of the mid-segment (12; 22; 32),
b) preparing of at least one textile attachment means in the vicinity of at least one of the two end segments (14; 24; 34),
**characterized in that** for preparing the at least one textile attachment means, during production of the strip at least one thread is led out of the developing textile structure in the vicinity of the at least one end segment (14; 24; 34), and subsequently the thread is led back into the textile structure of the strip thereby forming a mesh- or loop-like textile attachment means (16; 26; 36).

12. The method according to claim 11, **characterized in that** for preparing the at least one textile attachment means, at least one additional thread, particularly multiple threads, is/are introduced into the textile strip structure.

13. The method according to claim 11 or 12, **characterized in that** a strip is provided where the textile structure in the vicinity of at least one of the two end segments (24) has at least one separating thread (27) extending in the longitudinal direction of the strip, wherein preferably the at least one separating thread (27) is removed from the textile strip structure thereby forming a preferably slot-like opening (28).

14. The method according to any of the claims 11 to 13, **characterized in that** the at least one textile attachment means is stiffened, particularly heat-set, ultrasonically welded or stiffened by textile means, particularly twisted.

15. A surgical set, comprising a surgical implant according to any of the claims 1 to 10 and at least one surgical insertion device, preferably at least one surgical needle.

## Revendications

1. Implant chirurgical (10 ; 20 ; 30), de préférence pour l'utilisation pour traiter l'incontinence urinaire, sous la forme d'une bandelette de structure textile, avec une portion centrale (12 ; 22 ; 32) et deux portions d'extrémité (14 ; 24 ; 34) de chaque côté de la portion centrale (12 ; 22 ; 32), la bandelette présentant, dans la région d'au moins l'une des deux portions d'extrémité (14 ; 24 ; 34), au moins un moyen de fixation textile pour la fixation de la bandelette à un instrument chirurgical d'insertion, de préférence à une aiguille chirurgicale, **caractérisé en ce que** l'au moins un moyen de fixation textile présente au moins un fil qui participe à la réalisation de la structure textile de la bandelette.

2. Implant chirurgical (10 ; 20 ; 30) selon la revendication 1, **caractérisé en ce que** la bandelette présente, dans la région de deux portions d'extrémité (14 ; 24 ; 34), à chaque fois au moins un moyen de fixation textile.

3. Implant chirurgical (10 ; 20 ; 30) selon la revendication 1 ou 2, **caractérisé en ce que** l'au moins un moyen de fixation textile présente plusieurs fils qui participent à la réalisation de la structure textile de la bandelette.

4. Implant chirurgical (10 ; 20 ; 30) selon l'une quelconque des revendications précédentes, **caractérisé en ce que** l'au moins un moyen de fixation textile est réalisé sous forme de boucle de fil (16 ; 26 ; 36), notamment de boucle de velours et/ou de maille.

5. Implant chirurgical (10 ; 20 ; 30) selon l'une quelconque des revendications précédentes, **caractérisé en ce que** l'au moins un moyen de fixation textile comprend au moins un fil supplémentaire introduit dans la structure de bandelette, notamment plusieurs fils supplémentaires introduits dans la structure de bandelette.

6. Implant chirurgical (10 ; 20 ; 30) selon l'une quelconque des revendications précédentes, **caractérisé en ce que** l'au moins un moyen de fixation textile est réalisé sous forme de structure textile, sous forme de torsade ou de treillis, notamment sous forme de treillis rond ou en boucle.

7. Implant chirurgical (20) selon l'une quelconque des revendications précédentes, **caractérisé en ce qu'**au moins une portion d'extrémité (24), de préférence les deux portions d'extrémité (24), présentent une ouverture en forme de fente (28), et l'ouverture (28) est formée de préférence en enlevant de la structure textile de la bandelette au moins un fil de séparation ou de coupure (27) s'étendant dans la direction longitudinale de l'au moins une portion d'extrémité (24).

8. Implant chirurgical (10 ; 20 ; 30) selon l'une quelconque des revendications précédentes, **caractérisé en ce que** la bandelette est réalisée sous forme de filet textile, notamment sous forme de filet composite textile, de préférence sous forme de tissu à mailles, en particulier sous forme de tissu à mailles jetées.

9. Implant chirurgical (10 ; 20 ; 30) selon l'une quelconque des revendications précédentes, **caractérisé en ce que** la bandelette, notamment la portion centrale (12 ; 22 ; 32), possède une structure textile tridimensionnelle, de préférence avec laquelle des cellules corporelles peuvent entrer en prise par l'arrière, de préférence sous forme de velours, notamment de double velours.

10. Implant chirurgical (10 ; 20 ; 30) selon l'une quelconque des revendications précédentes, **caractérisé en ce que** la bandelette est réalisée sous forme d'armure tricotée, d'armure satin tricotée, d'armure velours, d'armure toile, d'armure pour tissu éponge, ou de combinaisons de celles-ci.

11. Procédé de fabrication d'un implant chirurgical (10 ; 20 ; 30) selon l'une quelconque des revendications précédentes, comprenant les étapes suivantes :
a) fourniture d'une bandelette à structure textile, avec une portion centrale (12 ; 22 ; 32) et deux portions d'extrémité (14 ; 24 ; 34) de chaque côté de la portion centrale (12 ; 22 ; 32),
b) réalisation d'au moins un moyen de fixation textile dans la région d'au moins l'une des deux portions d'extrémité (14 ; 24 ; 34),
**caractérisé en ce que** pour la réalisation de l'au moins un moyen de fixation textile lors de la fabrication de la bandelette, au moins un fil est ressorti de la structure textile en formation, dans la région de l'au moins une portion d'extrémité (14 ; 24 ; 34), et ensuite est réintroduit en réalisant un moyen de fixation textile (16 ; 26 ; 36) de préférence en forme de boucle, dans la structure textile de la bandelette.

12. Procédé selon la revendication 11, **caractérisé en ce que** pour réaliser l'au moins un moyen de fixation textile, au moins un fil supplémentaire, notamment plusieurs fils, sont introduits dans la structure de bandelette textile.

13. Procédé selon la revendication 11 ou 12, **caractérisé en ce qu'**une bandelette est fournie, dont la structure textile, dans la région d'au moins l'une des deux portions d'extrémité (24), présente au moins un fil de séparation (27) s'étendant dans la direction longitudinale de la bandelette, de préférence l'au moins un fil de séparation (27) étant enlevé de la structure de bandelette textile en créant une ouverture (28) de préférence en forme de fente.

14. Procédé selon l'une quelconque des revendications 11 à 13, **caractérisé en ce que** l'au moins un moyen de fixation textile est rigidifié, notamment thermofixé, soudé aux ultrasons, ou renforcé par un textile, notamment torsadé.

15. Kit chirurgical, comprenant un implant chirurgical selon l'une quelconque des revendications 1 à 10, et au moins un instrument d'insertion chirurgical, de préférence au moins une aiguille chirurgicale.
